# EUROPÄISCHE PATENTSCHRIFT

(11) **EP 4 204 805 B1**
(45) Veröffentlichungstag und Bekanntmachung des Hinweises auf die Patenterteilung: **11.09.2024**
(21) Anmeldenummer: 21790407.7
(22) Anmeldetag: 06.10.2021
(51) Int. Cl.: G01N 33/28, B01D 19/00

(54) **VORRICHTUNG UND VERFAHREN ZUM ENTGASEN EINES ENTGASUNGSGEFÄSS UND ENTSPRECHENDES PRÜFSYSTEM ZUR GASANALYSE**
APPARATUS AND METHOD FOR DEGASSING A DEGASSING VESSEL, AND CORRESPONDING TEST SYSTEM FOR GAS ANALYSIS
APPAREIL ET PROCÉDÉ DE DÉGAZAGE D'UN NAVIRE DE DÉGAZAGE, ET SYSTÈME DE TEST CORRESPONDANT POUR ANALYSE DE GAZ

(30) Priorität: 19.10.2020 AT 508952020; 23.11.2020 AT 510152020
(43) Veröffentlichungstag der Anmeldung: 05.07.2023
(73) Patentinhaber: Omicron electronics GmbH, 6833 Klaus (AT)
(72) Erfinder: GISELBRECHT, Dietmar, 6900 Bregenz (AT); ANGLHUBER, Martin, 6800 Feldkirch (AT)
(74) Vertreter: Kraus & Lederer PartGmbB
(86) Internationale Anmeldenummer: PCT/EP2021/077564
(87) Internationale Veröffentlichungsnummer: WO 2022/084041

(56) Entgegenhaltungen:
- CN-U- 210 674 325
- DE-A1- 102017 126 136
- NL-A- 7 901 929
- US-A- 4 407 665
- US-A- 4 456 172
- US-A- 4 763 514

## Beschreibung

### GEBIET DER ERFINDUNG

Die vorliegende Erfindung betrifft eine Vorrichtung und ein Verfahren zum Entgasen einer Einrichtung, wobei ein Totvolumenanteil reduziert wird, um beispielsweise gelöste Gase besser analysieren zu können.

### HINTERGRUND DER ERFINDUNG

Die DE 10 2017 126 136 A1 betrifft eine Vorrichtung zur Entgasung von Flüssigkeiten umfassend ein Entgasungssystem, wobei das Entgasungssystem eine erste Entgasungskammer, eine zweite Entgasungskammer, einen Flüssigkeitsvorrat, eine Pumpe sowie eine Zufuhrleitung aufweist, die den Flüssigkeitsvorrat mit der ersten Entgasungskammer verbindet, wobei die Pumpe saugseitig mit der ersten Entgasungskammer und druckseitig mit der zweiten Entgasungskammer verbunden ist, wobei das Entgasungssystem des Weiteren eine absperrbare Rückführungsleitung aufweist, die die beiden Entgasungskammern miteinander verbindet, und wobei die Vorrichtung einen Kontroller umfasst, der ausgebildet ist, das Entgasungssystem in einem ersten Betriebsmodus und in einem zweiten Betriebsmodus zu betreiben, wobei in dem ersten Betriebsmodus das Entgasungssystem derart geschaltet ist, dass die Pumpe Flüssigkeit aus der ersten Entgasungskammer abführt und einer Abnahmeeinheit für entgaste Flüssigkeit zuführt, und wobei das Entgasungssystem in dem zweiten Betriebsmodus derart geschaltet ist, dass Flüssigkeit durch die Rückführungsleitung aus der zweiten in die erste Entgasungskammer rückgeführt wird.

Beispielsweise zur Zustandsbestimmung und Fehlererkennung von Öl-Papier-isolierten Leistungstransformatoren ist die Analyse von in einem Isolieröl gelösten Gasen (Dissolved Gas Analysis (DGA)) ein wichtiges Vorgehen. Dabei müssen die zu analysierenden Gase vor der Analyse z.B. aus dem Isolieröl gelöst werden, was als Entgasung bekannt ist. Eine solche Entgasung kann durch unterschiedliche Verfahren erfolgen. Die höchste Extraktionsrate wird dabei mit einer vollständigen Entgasung mittels Vakuum erzielt.

Die Extraktion des Gases z.B. aus dem Isolieröl ist umso effektiver, je geringer der Druck in dem das Gas aufnehmenden Gefäß bzw. Volumen ist. Auch Entgasungspumpen, mit denen eine solche Extraktion des Gases vorgenommen wird, arbeiten umso effizienter je niedriger der Differenzdruck zwischen dem Druck eingangs der Pumpe und dem Druck ausgangs der Pumpe ist. Daher wird das Volumen auf der Ausgangsseite der Pumpe nach dem Stand der Technik so gewählt, dass der Druck in diesem Volumen bei der Entgasung oder Extraktion nicht zu sehr ansteigt.

Dazu können Teile einer Entgasungseinheit mittels einer Pumpe, welche auch für die Entgasung verwendet wird, evakuiert werden. Dabei besteht allerdings ein Problem, dass am Auslass der Pumpe und in den davon wegführenden Leitungen Umgebungsdruck herrscht, so dass diese im so genannten Totvolumen befindliche Luft nach dem Umschalten der Entgasungseinheit von der Evakuierung auf die Entgasung die Entgasungseinheit kontaminiert. Beispielsweise verursacht bereits ein Totvolumen von z.B. 100 µl bei einem Gesamtvolumen von 10 ml eine Kontamination von fast 7.000 ppm an Stickstoff, was Messungen von Stickstoff im niedrigen ppm-Bereich erschweren.

### ZUSAMMENFASSUNG DER ERFINDUNG

Daher stellt sich die vorliegende Erfindung die Aufgabe, eine Kontamination durch das in dem Totvolumen befindliche Gas (insbesondere Luft) im Vergleich zum Stand der Technik zu verringern. Dabei sollen die Mittel zur Lösung dieses Problems möglichst preiswert sein und möglichst wenig Platz benötigen.

Erfindungsgemäß wird diese Aufgabe durch eine Vorrichtung zur Entgasung eines Entgasungsgefäßes nach Anspruch 1, durch ein Prüfsystem nach Anspruch 12 und durch ein Verfahren zur Entgasung eines Entgasungsgefäßes nach Anspruch 13 gelöst. Die abhängigen Ansprüche definieren bevorzugte und/oder vorteilhafte Ausführungsformen der vorliegenden Erfindung.

Im Rahmen der vorliegenden Erfindung wird eine Vorrichtung zur Entgasung eines mit einer zu entgasenden Flüssigkeit befüllbaren Entgasungsgefäßes bereitgestellt. Diese Vorrichtung umfasst eine Steuereinrichtung, eine Pumpe, ein erstes Volumen oder Gefäß, ein zweites Volumen oder Gefäß, Ventilmittel, ein erstes Ventil und ein zweites Ventil. Die Ventilmittel können ein 3-Wege-Ventil oder zwei Ventile umfassen. Dabei ist die Pumpe zum einen über die Ventilmittel mit einem Luftauslass verbunden, über den beispielsweise die Vorrichtung und das zu entgasende Entgasungsgefäß evakuiert werden. Zum anderen ist die Pumpe ausgangsseitig über die Ventilmittel mit dem ersten Volumen verbunden, um so beispielsweise Gas aus dem zu entgasenden Entgasungsgefäß in das erste Volumen zu pumpen. Das erste Volumen und das zweite Volumen sind mittels des ersten Ventils miteinander verbunden, so dass abhängig von dem Zustand (geöffnet bzw. geschlossen) des ersten Ventils das erste Volumen mit dem zweiten Volumen verbunden ist oder die beiden Volumen getrennt sind. Eingangsseitig ist die Pumpe zum einen über ein zweites Ventil mit dem ersten Volumen verbunden, so dass die Pumpe (z.B. bei der Evakuierung) Gas aus dem zweiten Volumen herauspumpen kann, wenn das zweite Ventil (und das erste Ventil) geöffnet ist (sind). Zum anderen ist die Pumpe eingangsseitig mit dem zu entgasenden Entgasungsgefäß verbindbar, so dass die Pumpe Gas von oder aus dem Entgasungsgefäß weg pumpen kann, wenn die Pumpe mit dem Entgasungsgefäß verbunden ist.

Die Vorrichtung ermöglicht vorteilhafterweise, dass die Pumpe neben der Gasextraktion bzw. Entgasung sowohl zur Evakuierung des Entgasungsgefäßes als auch zur Evakuierung des ersten und des zweiten Volumens verwendet wird. Beim Evakuieren wird demnach auch das zweite Volumen auf einen sehr geringen Druck (z.B. 0,1 mbar) evakuiert. Wenn nach dem Evakuieren das erste Volumen und damit auch das mit dem ersten Volumen verbundene zweite Volumen wieder an den Ausgang der Pumpe geschaltet wird, verteilt sich die im Totvolumen befindliche Luft auf das Gesamtvolumen, welches von dem Totvolumen, dem ersten Volumen und dem zweiten Volumen gebildet wird. Durch das große zweite Volumen ist die dadurch in dem ersten Volumen vorhandene Menge an kontaminierender Luft deutlich geringer, als dies ohne das zweite Volumen der Fall wäre. Grob gesagt reduziert ein zweites Volumen, das einen um den Faktor X größeren Volumeninhalt als das erste Volumen aufweist, die Kontamination aufgrund der Luft im Totvolumen in dem ersten Volumen im Vergleich zu einer konventionellen Vorrichtung ohne das zweite Volumen um den Faktor X. Für die an die Evakuierung anschließende Entgasung wird das zweite Volumen von dem ersten Volumen mittels des ersten Ventils abgetrennt.

Indem erfindungsgemäß eine Kontamination durch die Luft im Totvolumen im Vergleich zum Stand der Technik stark reduziert werden kann, ist es vorteilhafterweise auch recht einfach möglich, ein Isolieröl mit guten Analyseergebnissen auf Sauerstoff und Stickstoff (Stoffe, die auch im Totvolumen vorhanden sind) zu untersuchen, da das untersuchte Gas nicht durch die Stoffe im Totvolumen dominiert wird.

Vorzugsweise ist der Volumeninhalt des zweiten Volumens zumindest zehnmal, besser zumindest 100-mal, noch besser zumindest 1000-mal größer als der Volumeninhalt des ersten Volumens.

Je größer das zweite Volumen im Verhältnis zu dem ersten Volumen ist, desto geringer ist vorteilhafterweise die Kontamination in dem ersten Volumen aufgrund der Luft in dem Totvolumen.

Das zweite Volumen, welches auch Gasreservoir genannt werden kann, kann durch einen günstigen Druckluftzylinder realisiert werden. Darüber hinaus kann das zweite Volumen bzw. das Gasreservoir an einer beliebigen Stelle in der Vorrichtung angeordnet werden. Etwaige längere Leitungen zu dem zweiten Volumen erhöhen nur dessen Volumen und steigern dadurch vorteilhafterweise den Effekt einer Verringerung der Kontamination durch die Luft im Totvolumen.

Des Weiteren ist es von Vorteil die Vorrichtung so auszugestalten, dass das Totvolumen möglichst klein gehalten wird, indem beispielsweise der Abstand zwischen dem Ausgang der Pumpe und den Ventilmitteln möglichst klein ist.

Die Vorrichtung schaltet mit Hilfe ihrer Steuereinrichtung die Ventilmittel derart, dass die Ventilmittel den Ausgang der Pumpe nur mit dem Luftauslass verbinden. Zusätzlich öffnet die Vorrichtung mit Hilfe ihrer Steuereinrichtung das erste Ventil und das zweite Ventil. Nachdem die Ventilmittel den Ausgang der Pumpe nur mit dem Luftauslass verbinden und das erste und das zweite Ventil geöffnet sind, steuert die Steuereinrichtung die Pumpe an, um Gas aus dem an die Vorrichtung angeschlossenen Entgasungsgefäß sowie aus dem ersten Volumen und dem zweiten Volumen zu pumpen, um das Entgasungsgefäß, das erst Volumen und das zweite Volumen zu evakuieren.

Indem die Ventilmittel den Ausgang der Pumpe nur mit dem Luftauslass (und nicht noch mit dem ersten Volumen) verbinden und das erste sowie das zweite Ventil geöffnet sind, kann die Pumpe vorteilhafterweise effektiv in einem Vorgang (d.h. z.B. ohne ein Umschalten von Ventilen) sowohl das mit der Vorrichtung verbundene Entgasungsgefäß als auch das erste und das zweite Volumen evakuieren.

Gemäß einer erfindungsgemäßen Ausführungsform schaltet die Vorrichtung mit Hilfe ihrer Steuereinrichtung die Ventilmittel derart, dass die Ventilmittel den Ausgang der Pumpe direkt nur mit dem ersten Volumen verbinden. Darüber hinaus öffnet die Vorrichtung mit Hilfe ihrer Steuereinrichtung das erste Ventil und das zweite Ventil, so dass es anschließend zu einem Druckausgleich in einem Gesamtvolumen kommt, welches das erste Volumen, das zweite Volumen und das Totvolumen umfasst.

Indem bei dem Druckausgleich das erste Volumen und das zweite Volumen über das erste Ventil miteinander verbunden sind, verteilt sich die in dem Totvolumen vorhandene Luft vorteilhafterweise nicht nur in dem ersten Volumen, sondern auch in dem größeren zweiten Volumen. Dadurch ist die Kontamination in dem ersten Volumen vorteilhafterweise deutlich geringer, als wenn das zweite Volumen nicht vorhanden wäre, wie es nach dem Stand der Technik der Fall ist.

Gemäß einer erfindungsgemäßen Ausführungsform schaltet die Vorrichtung mit Hilfe ihrer Steuereinrichtung die Ventilmittel derart, dass die Ventilmittel den Ausgang der Pumpe direkt nur mit dem ersten Volumen verbinden. Zusätzlich schließt die Vorrichtung mittels ihrer Steuereinrichtung das erste Ventil und das zweite Ventil. Nachdem das erste Ventil und das zweite Ventil geschlossen sind, steuert die Steuereinrichtung die Pumpe an, um Gas von dem Entgasungsgefäß in das erste Volumen zu pumpen.

Indem die Ventilmittel die Pumpe ausgangsseitig nur mit dem ersten Volumen verbinden und das erste und das zweite Ventil geschlossen sind, kann die Vorrichtung vorteilhafterweise auch dafür eingesetzt werden, um Gas von dem Entgasungsgefäß mittels der Pumpe in das erste Volumen zu entgasen. Demnach wird vorteilhafterweise dieselbe Pumpe sowohl für die Evakuierung als auch für die Entgasung eingesetzt.

Gemäß einer erfindungsgemäßen Ausführungsform umfasst die Vorrichtung ein drittes Ventil. Über dieses dritte Ventil ist das zu entgasende Entgasungsgefäß mit dem Eingang der Pumpe verbunden.

Das dritte Ventil ermöglicht, dass die Vorrichtung dauerhaft (über das dritte Ventil) mit dem zu entgasenden Entgasungsgefäß verbunden ist. Mit diesem dritten Ventil ist es sehr einfach, den Evakuierungsschritt, den Druckausgleich und auch den Entgasungsschritt zu steuern. Bei dem Evakuierungsschritt und Entgasungsschritt ist das dritte Ventil geöffnet, so dass die Pumpe Gas von dem zu entgasenden Entgasungsgefäß wegpumpen kann. Dagegen ist das dritte Ventil bei dem Druckausgleich sowie bei einem eventuellen Komprimierungsschritt (siehe unten) geschlossen, wie es im Folgenden noch genauer beschrieben wird.

Gemäß einer erfindungsgemäßen Ausführungsform schaltet die Vorrichtung mittels ihrer Steuereinrichtung die Ventilmittel derart, dass die Ventilmittel den Ausgang der Pumpe nur mit dem Luftauslass verbinden. Zusätzlich werden das erste, zweite und dritte Ventil mittels der Steuereinrichtung geöffnet. Nachdem das erste, zweite und dritte Ventil geöffnet sind, steuert die Steuereinrichtung die Pumpe an, wodurch die Pumpe Gas aus dem Entgasungsgefäß, dem ersten Volumen und dem zweiten Volumen pumpt, so dass das Entgasungsgefäß, das erste Volumen und das zweite Volumen evakuiert werden.

Da der Ausgang der Pumpe nur mit dem Luftauslass verbunden ist und das erste bis dritte Ventil geöffnet sind, ist die Pumpe vorteilhafterweise in der Lage, in einem Schritt das zu entgasende Entgasungsgefäß sowie das erste und das zweite Volumen zu evakuieren.

Gemäß einer erfindungsgemäßen Ausführungsform schaltet die Vorrichtung mit Hilfe ihrer Steuereinrichtung die Ventilmittel derart, dass die Pumpe mit ihrem Ausgang direkt nur mit dem ersten Volumen verbunden ist. Zusätzlich werden mittels der Steuereinrichtung das erste Ventil und das zweite Ventil geöffnet sowie das dritte Ventil geschlossen. Sobald das erste Ventil und das zweite Ventil geöffnet und das dritte Ventil geschlossen sind, wird ein Druckausgleich in einem Gesamtvolumen durchgeführt, welches das erste Volumen, das zweite Volumen und das Totvolumen umfasst.

Da während des Druckausgleichs das erste Volumen, das zweite Volumen und das Totvolumen ein Gesamtvolumen ausbilden, verteilt sich die in dem Totvolumen vorhandene Luft vorteilhafterweise in diesem Gesamtvolumen. Dadurch ist die durch die Luft in dem Totvolumen stattfindende Kontamination in dem ersten Volumen vorteilhafterweise deutlich geringer, als wenn das zweite Volumen nicht vorhanden wäre, wie es bei einer konventionellen Vorrichtung der Fall ist.

Gemäß einer erfindungsgemäßen Ausführungsform schaltet die Vorrichtung mittels ihrer Steuereinrichtung die Ventilmittel derart, dass die Ventilmittel den Ausgang der Pumpe direkt nur mit dem ersten Volumen verbinden. Darüber hinaus werden mittels der Steuereinrichtung das erste und das zweite Ventil geschlossen und das dritte Ventil geöffnet. Nachdem das erste und das zweite Ventil geschlossen und das dritte Ventil geöffnet sind, steuert die Steuereinrichtung die Pumpe an, so dass die Pumpe Gas von dem Entgasungsgefäß in das erste Volumen pumpt.

Da die Ventilmittel die Pumpe ausgangsseitig nur mit dem ersten Volumen verbinden und das erste und das zweite Ventil geschlossen sind, wird das Gas aus dem zu entgasenden Entgasungsgefäß über das geöffnete dritte Ventil nur in das erste Volumen entgast bzw. gepumpt. Dieselbe Pumpe kann demnach vorteilhafterweise sowohl für die Evakuierung des Entgasungsgefäß und der Vorrichtung wie auch für die Entgasung eingesetzt werden.

Gemäß einer erfindungsgemäßen Ausführungsform umfasst die Vorrichtung auch einen Sensor. Dabei ist der Sensor zumindest teilweise innerhalb des ersten Volumens angeordnet und ausgestaltet, um ein Gas in dem ersten Volumen zu analysieren.

Mit Hilfe des Sensors kann die Analyse des Gases in dem ersten Volumen nach der Entgasung quasi automatisiert erfolgen. Dieser Sensor misst die Gase. Bei dem Sensor kann es sich beispielsweise um einen Halbleitersensor, einen optischen Sensor (oder eine optische Messapparatur), einen Wärmeleitfähigkeitssensor oder um einen chemischen Analyseapparat (z.B. einen Gaschromatograph) handeln. Mit anderen Worten ist der Sensor insbesondere irgendeine Vorrichtung, die Gase messen kann und - in diesem Fall - diese Gase in einer möglichst hohen (absoluten) Konzentration benötigt.

Gemäß einer erfindungsgemäßen Ausführungsform umfasst das erste Volumen ein erstes Teilvolumen und ein zweites Teilvolumen. Dabei ist das erste Teilvolumen über ein weiteres Ventil der Vorrichtung mit dem zweiten Teilvolumen verbunden. Mittels der Steuereinrichtung werden die Ventilmittel derart geschaltet, dass sie den Ausgang der Pumpe direkt nur mit dem ersten Volumen bzw. ersten Teilvolumen verbinden. Darüber hinaus schließen die Steuermittel das erste, das dritte und das weitere Ventil, während sie das zweite Ventil öffnen. Nachdem das erste, das dritte und das weitere Ventil geschlossen sind und das zweite Ventil geöffnet ist, wird die Pumpe von der Steuereinrichtung angesteuert, um anschließend Gas von dem zweiten Teilvolumen in das erste Teilvolumen zu pumpen. Dadurch wird vorteilhafterweise das von dem zu entgasenden Entgasungsgefäß entgaste Gas in dem ersten Teilvolumen komprimiert, wodurch die Analyse dieses Gases erleichtert wird.

Bei dieser Ausführungsform ist vorteilhafterweise das weitere Ventil bei dem Entgasungsschritt geöffnet, so dass das Gas aus dem zu entgasenden Entgasungsgefäß sowohl in das erste als auch in das zweite Teilvolumen gepumpt wird. Bei dem anschließenden Komprimierungsschritt wird das weitere Ventil geschlossen, so dass es nur noch eine geöffnete Verbindung über die Pumpe zwischen dem ersten Teilvolumen und dem zweiten Teilvolumen gibt. Dadurch kann die Pumpe im Komprimierungsschritt Gas aus dem zweiten Teilvolumen (Niederdruckvolumen) in das erste Teilvolumen (Hochdruckvolumen) pumpen, um dort den Druck zu erhöhen. Durch den höheren Druck wird die Konzentration der Gase in dem ersten Teilvolumen erhöht, was eine anschließende Analyse dieser Gase begünstigt.

Die erfindungsgemäße Vorrichtung ermöglicht vorteilhafterweise, dass bei der Entgasung das extrahierte Gas in beide Teilvolumen gepumpt bzw. gefördert wird, so dass der Druck auf der Ausgangsseite der Pumpe nur geringfügig ansteigt. Nach der Entgasung kann dieselbe Pumpe das extrahierte Gas aus dem zweiten Teilvolumen in das erste Teilvolumen pumpen, wodurch sich der Druck des extrahierten Gases in dem ersten Teilvolumen erhöht.

Vorzugsweise ist das zweite Teilvolumen bzw. Niederdruckvolumen zumindest fünfmal, besser zehnmal, noch besser 20-mal größer als das erste Teilvolumen bzw. Hochdruckvolumen. Es ist allerdings auch möglich, dass (abhängig von der vorhandenen Gasmenge und der Pumpe) das zweite Teilvolumen bzw. Niederdruckvolumen 100-mal oder gar 1000-mal größer als das erste Teilvolumen bzw. Hochdruckvolumen ist.

Gemäß dieser Ausführungsform ist das Hochdruckvolumen deutlich kleiner als das Niederdruckvolumen. Das Volumen des Niederdruckvolumens ist dabei vorteilhafterweise so gewählt, dass der Druck auf der Ausgangsseite der Pumpe beim Entgasen nicht derart ansteigt, dass die Effizienz der Pumpe beeinträchtigt wird.

Bei einer beispielhaften Anwendung kann nur sehr wenig Gas (ca. 1 ml) zur Verfügung stehen. In diesem Fall kann das erste Teilvolumen (Hochdruckvolumen) 0,5 ml und das zweite Teilvolumen (Niederdruckvolumen) 10 ml groß sein.

Die absoluten Größen der Teilvolumen hängen in der Regel davon ab, wie viel Gas gemessen werden soll bzw. kann (d.h. zur Verfügung steht). Dabei können die absoluten Größen für die Teilvolumen auch von einem eingesetzten Sensor (zur Analyse des Gases) abhängen. Es ist durchaus möglich, dass die Volumen im µl-Bereich liegen (also deutlich kleiner als 1 ml sind). Auf der anderen Seite können auch mehrere Liter an Gas (z.B. bei Emissionsmessungen) auftreten, wobei die Teilvolumen dann entsprechend größer (im Bereich von 1 l bis 20 I) gewählt werden.

Die Verhältnisse zwischen dem Gasvolumen (Volumen des zu analysierenden Gases bzw. Entgasungsvolumen) und dem ersten und zweiten Teilvolumen sind im Wesentlichen ähnlich. Das zweite Teilvolumen (Niederdruckvolumen) wird meist gleich oder größer als das Gasvolumen gewählt, während das erste Teilvolumen (Hochdruckvolumen) deutlich kleiner ist. Die Verhältnisse können von der eingesetzten Pumpe abhängig sein. Beispielsweise kann beim Einsatz einer Hochdruckpumpe auch das zweite Teilvolumen (Niederdruckvolumen) nur z.B. ein Zehntel des Gasvolumens betragen. In diesem Fall wäre also das zweite Teilvolumen kleiner als das Gasvolumen.

Im Rahmen der vorliegenden Erfindung wird auch ein Prüfsystem zum Prüfen von gelösten Gasen und Gas an oder in einer Anlage, wie z.B. einer Hochspannungsanlage, bereitgestellt. Dabei umfasst das Prüfsystem eine Auswerteeinheit und eine erfindungsgemäße Vorrichtung zur Entgasung, wie sie vorab beschrieben ist. Das Prüfsystem ist ausgestaltet, um eine Analyse des Gases in oder von der Anlage (z.B. eine Analyse des in dem Isolieröl einer Hochspannungsanlage gelösten Gases) auszuführen. Dabei ist die Auswerteeinheit ausgestaltet, um, beispielsweise mit Hilfe des Sensors der Vorrichtung, das in das erste Volumen gepumpte Gas zu analysieren und um abhängig von dieser Analyse ein Ergebnis der Prüfung der Anlage zu erstellen und vorteilhafterweise auszugeben.

Das erfindungsgemäße Prüfsystem kann in ähnlicher Weise wie die erfindungsgemäße Vorrichtung an ölisolierten Hochspannungsanlagen, wie z.B. Leistungstransformatoren, Stromwandlern, Spannungswandlern und mittels Gas isolierten Schaltanlagen, eingesetzt werden. Bei dem zu analysierenden Gas kann es sich um ein Gas, welches zur Isolierung der Hochspannungsanlage selbst eingesetzt wird, oder um ein Gas, welches sich aus einer Flüssigkeit einer Isolierung oder einem Isolieröl gelöst hat, handeln.

Schließlich wird im Rahmen der vorliegenden Erfindung ein Verfahren zur Entgasung eines Entgasungsgefäßes bereitgestellt. Dieses Verfahren umfasst folgende Schritte:
- Verbinden eines ersten Volumens mit einem zweiten Volumen, welches von dem ersten Volumen abtrennbar ist. Dieser Schritt kann beispielsweise ausgeführt werden, indem ein Ventil zwischen dem ersten und dem zweiten Volumen geöffnet wird.
- Evakuieren des Entgasungsgefäßes zusammen mit dem ersten und dem zweiten Volumen. In diesem Schritt sind das zu entgasende Entgasungsgefäß sowie das erste und das zweite Volumen insbesondere über geöffnete Ventile miteinander verbunden, so dass dieselbe Pumpe das zu entgasende Entgasungsgefäß zusammen mit dem ersten und dem zweiten Volumen in einem Schritt evakuieren kann, ohne dass irgendwelche Einstellungsänderungen (z.B. die Änderung der Einstellung bestimmter Ventile oder Ventilmittel) vorgenommen werden müssen.
- Abtrennen des zu entgasenden Entgasungsgefäßes von dem ersten Volumen und dem zweiten Volumen. Dieser Schritt kann beispielsweise automatisiert durchgeführt werden, indem ein Ventil, mit welchem das zu entgasende Entgasungsgefäß mit der Vorrichtung verbunden ist, geschlossen wird.
- Ausführen eines Druckausgleichs in einem Gesamtvolumen, welches das erste Volumen, das zweite Volumen und das Totvolumen umfasst. In diesem Schritt existiert vorteilhafterweise keine Verbindung zwischen dem ersten Volumen oder dem zweiten Volumen und der Außenwelt. Dies kann beispielsweise bewerkstelligt werden, indem die vorab beschriebenen Ventilmittel derart geschaltet sind, dass weder der Ausgang der Pumpe noch das erste Volumen über die Ventilmittel mit dem Luftauslass verbunden sind.
- Trennen des zweiten Volumens von dem ersten Volumen. Dazu wird vorzugsweise das Ventil, welches das erste Volumen mit dem zweiten Volumen verbindet, geschlossen.
- Befüllen des Entgasungsgefäßes mit einer zu entgasenden Flüssigkeit.
- Entgasen des Entgasungsgefäßes in das erste Volumen. In diesem Schritt wird insbesondere das Gas von dem zu entgasenden Entgasungsgefäß mittels der Pumpe in das erste Volumen gepumpt.

Ein wichtiger Schritt des vorab beschriebenen Verfahrens ist der Schritt der Ausführung des Druckausgleichs. In diesem Schritt verteilt sich die Luft in dem Totvolumen auf das erste und das zweite Volumen. Da das zweite Volumen überhaupt vorhanden ist und deutlich größer ist als das erste Volumen, kontaminiert das Gas in dem Totvolumen das Gas in dem ersten Volumen in einem deutlich geringeren Ausmaß, als wenn das zweite Volumen nicht vorhanden wäre.

Darüber hinaus entsprechen die Vorteile des erfindungsgemäßen Verfahrens im Wesentlichen den Vorteilen der erfindungsgemäßen Vorrichtung, welche vorab beschrieben sind, so dass hier auf eine Wiederholung verzichtet wird.

In das Entgasungsgefäß wird beispielsweise manuell eine zu entgasende Flüssigkeit (z.B. Öl) gefüllt. Es ist allerdings auch möglich, dass kein manueller Vorgang erforderlich ist, indem beispielsweise die zu entgasende Flüssigkeit automatisch in das Entgasungsgefäß gefüllt (gepumpt) wird oder indem das der erfindungsgemäßen Vorrichtung oder dem erfindungsgemäßen Prüfsystem zugeführte und zu analysierende Gas direkt einer Anlage (z.B. einer Hochspannungsanlage) entnommen wird. In dem letzteren Fall entspricht das zu entgasende Entgasungsgefäß quasi der Anlage. Daher kann die Vorrichtung zur Entgasung einer Isolierflüssigkeit (z.B. Öl) einer Hochspannungsanlage ausgestaltet sein.

Neben der Überprüfung von Hochspannungsanlagen kann die vorliegende Erfindung auch allgemein für Gasmessgeräte eingesetzt werden. Damit kann die vorliegende Erfindung zur Qualitätskontrolle im Labor, zur Prozessanalyse und Prozessüberwachung eingesetzt werden für:
- Petrochemie- und Chemie-Anlagen
- Erdgas-Aufbereitungsanlagen
- Bio-Gas-Anlagen
- Brennwertbestimmungen bei online Erdgasanalysen und bei Energieerzeugungen
- Emissionsmessungen

### KURZE BESCHREIBUNG DER FIGUREN

Die Erfindung wird nachfolgend anhand bevorzugter Ausführungsformen und unter Bezugnahme auf die Zeichnungen näher erläutert.
In Fig. 1 ist schematisch eine erfindungsgemäße Vorrichtung dargestellt, welche mit einem zu entgasenden Entgasungsgefäß verbunden ist.
In Fig. 2 ist schematisch ein Prüfsystem dargestellt, welches mit einer zu prüfenden Hochspannungsanlage verbunden ist.

### DETAILLIERTE BESCHREIBUNG VON AUSFÜHRUNGSBEISPIELEN

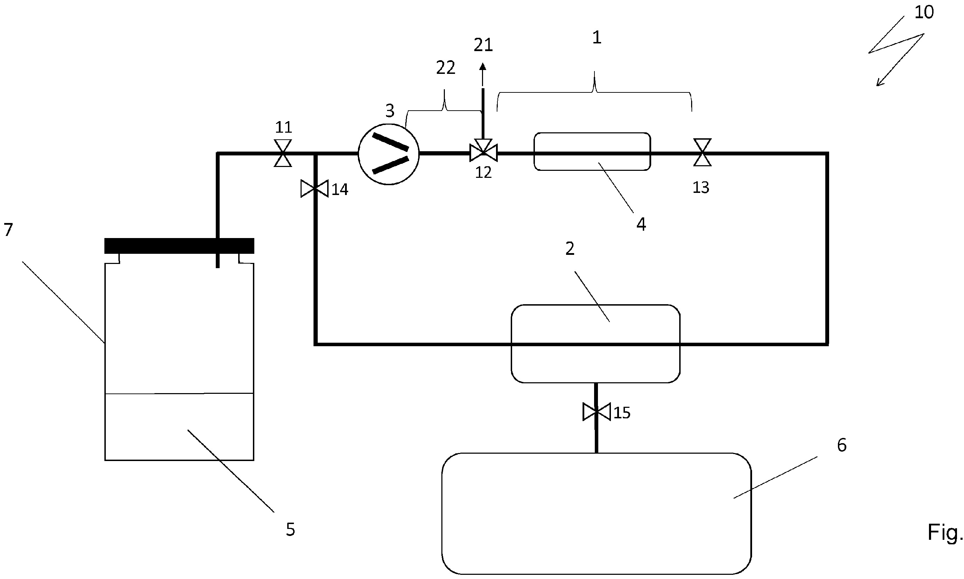

In Fig. 1 ist schematisch eine erfindungsgemäße Vorrichtung 10 dargestellt, welche mit einer zu entgasenden Einrichtung (hier einem Entgasungsvolumen bzw. Entgasungsgefäß 7) in Verbindung steht.

Die Vorrichtung umfasst ein 3-Wege-Ventil 12, ein erstes Ventil 15, ein zweites Ventil 14, ein drittes Ventil 11, ein weiteres Ventil 13, eine Pumpe 3, ein erstes Volumen, welches ein Sensorgefäß bzw. erstes Teilvolumen 1 und ein Entspannungsgefäß bzw. zweites Teilvolumen 2 umfasst, ein Gasreservoir bzw. zweites Volumen 6 und einen Sensor 4, der in dem ersten Volumen, genauer in dem ersten Teilvolumen 1 (Analysevolumen) angeordnet ist. Das erste Ventil 15 ist zwischen dem ersten Volumen (genauer zweiten Teilvolumen 2) und dem zweiten Volumen 6 angeordnet. Die Pumpe 3 ist so angeordnet, dass die Pumpe 3 eingangsseitig über das zweite Ventil 14 mit dem ersten Volumen (genauer zweiten Teilvolumen 2) und/oder über das dritte Ventil 11 mit dem Entgasungsgefäß 7 in Verbindung steht. Über das 3-Wege-Ventil 12 kann die Pumpe 3 ausgangsseitig entweder mit einem Luftauslass 21 oder mit dem ersten Volumen (genauer ersten Teilvolumen 1) verbunden werden. Das weitere Ventil 13 ist zwischen dem ersten Teilvolumen 1 (Analysevolumen) und dem zweiten Teilvolumen 2 (Entspannungsvolumen) angeordnet.

Die Vorrichtung 10 ist so konstruiert, dass das Totvolumen 22 (Volumen innerhalb der Leitung von dem Ausgang der Pumpe 3 bis zu dem Eingang des 3-Wege-Ventils 12) möglichst klein ist. Das Volumen des Gasreservoirs 6 orientiert sich insbesondere an der Summe aus dem Totvolumen 22, Analysevolumen 1 und Entspannungsvolumen 2, wird aber in der Regel auch durch die maximal praktisch mögliche Bauform begrenzt. Es gilt dabei prinzipiell: Je größer der Volumeninhalt des Gasreservoirs 6, desto besser ist es. Beispielsweise kann das Entgasungsvolumen einen Volumeninhalt von ca. 300 ml (ca. 700 ml) (abhängig von dem verwendeten Entgasungsgefäß 7), das Entspannungsvolumen 2 einen Volumeninhalt von ca. 10 ml, das Analysevolumen 1 einen Volumeninhalt von ca. 500 µl und das Gasreservoir 6 einen Volumeninhalt von ca. 750 ml (ca. 1,5 I) aufweisen.

Im Folgenden werden die Schritte anhand der in Fig. 1 dargestellten Vorrichtung 10 erläutert, die gemäß einer erfindungsgemäßen Ausführungsform von der Evakuierung bis zur Analyse der Gase durchgeführt werden.

Zu Beginn wird in einem Verbindungsschritt das erste Volumen 1, 2 mit dem zweiten Volumen 6 verbunden, indem das erste Ventil 15 geöffnet wird. Alternativ kann das Ergebnis dieses Verbindungsschritts (d.h. das geöffnete erste Ventil 15) quasi als Voraussetzung für den folgenden Evakuierungsschritt definiert sein.

Bei dem Evakuierungsschritt wird die Vorrichtung 10 und das Entgasungsgefäß 7 evakuiert. Dabei ist das 3-Wege-Ventil 12 derart geschaltet, dass es den Ausgang der Pumpe 3 nur mit dem Luftauslass 21 verbindet. Das erste Ventil 15, das zweite Ventil 14, das dritte Ventil 11 und das weitere Ventil 13 sind geöffnet. Dadurch pumpt die Pumpe 3 die Luft bzw. die Gase aus dem ersten Volumen 1, 2, dem zweiten Volumen 6 und dem Entgasungsgefäß 7 über den Luftauslass 21 aus der Vorrichtung 10 und dem Entgasungsgefäß 7 hinaus. Am Ende des Evakuierungsschritts bzw. der Evakuierungsphase weisen das Entgasungsgefäß 7, das erste Volumen 1, 2 und das zweite Volumen 6 beispielsweise einen Druck von 0,1 mbar auf, während der Teil der Vorrichtung 10 von dem Ausgang der Pumpe 3 bis zu dem Eingang des 3-Wege-Ventils 12 einen Umgebungsdruck (ca. 1 bar) aufweist. Dieser Teil bzw. dieses Volumen ist das Totvolumen 22. Die in diesem Totvolumen 22 befindliche Luft kontaminiert nach dem Umschalten von dem Evakuierungsschritt zu dem Entgasungsschritt insbesondere das Analysevolumen bzw. erste Teilvolumen 1, in dem der Sensor 4 angeordnet ist.

Nach dem Evakuierungsschritt wird das Entgasungsgefäß 7 von der Vorrichtung 10 abgetrennt, indem das dritte Ventil 11 geschlossen wird. Anschließend erfolgt ein Druckausgleich, indem das 3-Wege-Ventil 12 derart geschaltet wird, dass weder der Ausgang der Pumpe 3 noch das erste Volumen (genauer das erste Teilvolumen 1) eine Verbindung zu dem Luftauslass 21 haben. Insbesondere wird das 3-Wege-Ventil 12 so geschaltet, dass der Ausgang der Pumpe 3 nur mit dem ersten Volumen (genauer dem ersten Teilvolumen 1) verbunden ist. Der sich in Folge des Druckausgleichs einstellende Druck wird durch das Verhältnis der Volumengrößen des ersten Volumens 1, 2 und des zweiten Volumens 6 zu dem Totvolumen 22 bestimmt. Aufgrund der großen Volumengröße des zweiten Volumens bzw. Gasreservoirs 6, steigt der Druck in dem ersten Volumen 1, 2 und insbesondere in dem ersten Teilvolumen 1, in dem das Gas analysiert werden wird, deutlich weniger stark an, als wenn das Gasreservoir 6 nicht vorhanden wäre. Dadurch ist die Kontamination durch das Totvolumen 22 auch deutlich geringer. Bei einer Volumengröße des Analysevolumens 1 von 500 µl, einer Volumengröße des Entspannungsvolumens 2 von 10 ml und einer Volumengröße des Gasreservoirs 6 von 750 ml kann sich beispielsweise ein Druck von 0,6 mbar in dem ersten Volumen 1, 2 und dem zweiten Volumen 6 einstellen. Der Druck in dem abgeschlossenen Entgasungsgefäß 7 bleibt bei 0,1 mbar.

Nachdem sich der Druck gleichmäßig innerhalb der Volumen 1, 2, 6 der Vorrichtung 10 verteilt hat, wird das zweite Volumen bzw. Gasreservoir 6 abgetrennt, indem das erste Ventil 6 geschlossen wird.

Darüber hinaus wird vorteilhafterweise das Entgasungsgefäß 7 mit der zu entgasenden Flüssigkeit (z.B. Öl 5) befüllt.

Das Abtrennen des Gasreservoirs 6 und das Befüllen des Entgasungsgefäßes 7 können in einer beliebigen Reihenfolge ausgeführt werden, Es ist auch möglich dass diese beiden Schritte gleichzeitig ausgeführt werden.

Nach dem Abtrennen des zweiten Volumens bzw. Gasreservoirs 6 und dem Befüllen des Entgasungsgefäßes 7 wird in einem Entgasungsschritt das zu entgasende Gas der Flüssigkeit 5 in die Vorrichtung 10 entgast bzw. gepumpt. Dazu wird das dritte Ventil 11 geöffnet und das zweite Ventil 14 geschlossen. Das 3-Wege-Ventil 12 verbindet weiter den Ausgang der Pumpe 3 nur mit dem ersten Volumen (genauer ersten Teilvolumen 1), das weitere Ventil 13 ist geöffnet und verbindet weiter das erste Teilvolumen 1 mit dem Entspannungsvolumen bzw. zweiten Teilvolumen 2, während das erste Ventil 15 weiter geschlossen ist. Anschließend wird die Pumpe 3 betätigt, welche das Gas aus dem Entgasungsgefäß 7 in das erste Volumen 1, 2 pumpt.

Optional kann zwischen dem Entgasungsschritt und dem folgenden Messschritt noch ein Komprimierungsschritt erfolgen, um die Gase in dem ersten Teilvolumen bzw. Analysevolumen 1 zu komprimieren. Für diesen Komprimierungsschritt bleibt das erste Ventil 15 geschlossen, und das 3-Wege-Ventil 12 verbindet weiter den Ausgang der Pumpe 3 nur mit dem ersten Teilvolumen 1. Das dritte Ventil 11 und das weitere Ventil 13 werden geschlossen, während das zweite Ventil 14 geöffnet wird. Anschließend pumpt die Pumpe 3 die Gase aus dem zweiten Teilvolumen bzw. Entspannungsvolumen 2 in das erste Teilvolumen 1, wodurch die Gase in dem ersten Teilvolumen bzw. Analysevolumen 1 komprimiert werden, was eine Analyse der Gase mittels des Sensors 4 in dem Analysevolumen 1 verbessert. Je mehr die Gase in dem Analysevolumen 1 komprimiert sind, desto höher ist die Konzentration bestimmter zu analysierender Teilchen in den Gasen, was vorteilhafterweise eine Analysefähigkeit des Sensors 4 erhöht.

Es ist festzuhalten, dass es die vorliegende Erfindung ermöglicht, dass der Evakuierungsschritt, der Entgasungsschritt und der Komprimierungsschritt mit ein und derselben Pumpe 3 ausgeführt werden. Insbesondere wird keine weitere Pumpe benötigt, um Luft aus dem Totvolumen 22 am Ausgang der Pumpe 3 wegzupumpen.

Nach dem Entgasungsschritt oder optional nach dem Komprimierungsschritt erfolgt ein Messschritt, in dem die Gase in dem ersten Teilvolumen bzw. Analysevolumen 1 mit Hilfe des Sensors 4 analysiert werden.

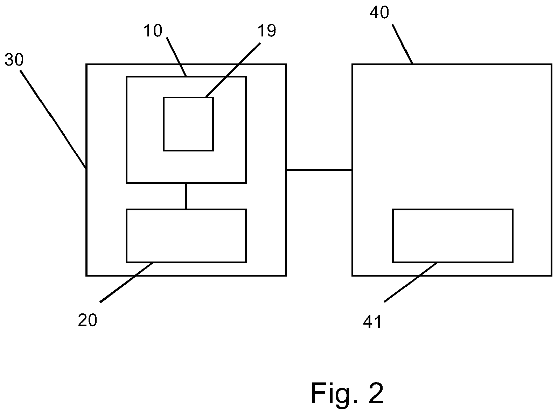

Die Durchführung des Entgasungsvorgangs bzw. Entgasungsschritts und die Ansteuerung der Ventile 11-15 sowie der Pumpe 3 erfolgt vorzugsweise automatisch bzw. rechnergestützt mittels einer geeigneten Steuereinrichtung (vgl. die in Fig. 2 gezeigte Steuereinrichtung 19).

In Fig. 2 sind schematisch ein erfindungsgemäßes Prüfsystem 30 und eine Hochspannungsanlage 40 dargestellt. Dabei ist das Prüfsystem 30 ausgestaltet, um eine Isolierung 41 der Hochspannungsanlage 40 zu überprüfen. Das Prüfsystem 30 umfasst eine erfindungsgemäße Vorrichtung 10 zur Entgasung, wie sie vorab beschrieben und in Fig. 1 schematisch dargestellt ist. Darüber hinaus umfasst das Prüfsystem 30 eine Auswerteeinheit 20, um abhängig von der durch die Vorrichtung 10 mit ihrer Steuereinrichtung 19 ausgeführten Evakuierung, Entgasung, optionalen Komprimierung und anschließenden Analyse des Gases ein Ergebnis der Überprüfung zu erstellen. Dabei analysiert die Vorrichtung 10 ein aus der Isolierung 41 kommendes Gas, wobei anhand der Analyse dieses Gases die Qualität der Isolierung 41 und damit ein Maß für die Einsatzbereitschaft der Hochspannungsanlage 40 selbst bestimmt werden kann.

## Patentansprüche

1. Vorrichtung zur Entgasung eines mit einer zu entgasenden Flüssigkeit befüllbaren Entgasungsgefäßes (7),
mit einer Pumpe (3), die zum Pumpen von Gas ausgestaltet und ausgangsseitig zum einen über Ventilmittel (12) mit einem Luftauslass (21) und zum anderen über die Ventilmittel (12) mit einem ersten Volumen (1, 2) verbunden ist,
wobei das erste Volumen (1, 2) und ein zweites Volumen (6) über ein erstes Ventil (15) verbunden sind,
wobei die Pumpe (3) eingangsseitig zum einen über ein zweites Ventil (14) mit dem ersten Volumen (1, 2) verbunden ist und zum anderen mit dem zu entgasenden Entgasungsgefäß (7) verbindbar ist, und
mit einer Steuereinrichtung (19) zum Ansteuern der Pumpe (3), der Ventilmittel (12), des ersten Ventils (15) und des zweiten Ventils (14),
wobei die Vorrichtung (10) ausgestaltet ist, um mittels der Steuereinrichtung (19) die Ventilmittel (12) derart zu schalten, dass die Ventilmittel (12) die Pumpe (3) ausgangsseitig nur mit dem Luftauslass (22) verbinden, um das erste Ventil (15) und das zweite Ventil (14) zu öffnen und um anschließend mittels der Pumpe (3) das Entgasungsgefäß (7), das erste Volumen (1, 2) und das zweite Volumen (6) zu evakuieren.

2. Vorrichtung nach Anspruch 1,
**dadurch gekennzeichnet,**
**dass** das zweite Volumen (6) zumindest zehnmal größer als das erste Volumen (1, 2) ist.

3. Vorrichtung nach einem der vorhergehenden Ansprüche,
**dadurch gekennzeichnet,**
**dass** die Vorrichtung (10) ausgestaltet ist, um mittels der Steuereinrichtung (19) die Ventilmittel (12) derart zu schalten, dass die Ventilmittel (12) die Pumpe (3) ausgangsseitig nur mit dem ersten Volumen (1, 2) verbinden und um das erste Ventil (15) und das zweite Ventil (14) zu öffnen, so dass anschließend ein Druckausgleich in einem Gesamtvolumen, welches von dem ersten Volumen (1, 2) und dem zweiten Volumen (6) sowie einem Totvolumen (22) gebildet wird, ausgeführt wird.

4. Vorrichtung nach einem der vorhergehenden Ansprüche,
**dadurch gekennzeichnet,**
**dass** die Vorrichtung (10) ausgestaltet ist, um mittels der Steuereinrichtung (19) die Ventilmittel (12) derart zu schalten, dass die Ventilmittel (12) die Pumpe (3) ausgangsseitig nur mit dem ersten Volumen (1, 2) verbinden und um das erste Ventil (15) und das zweite Ventil (14) zu schließen und um anschließend mittels der Pumpe (3) Gas von der Einrichtung (7) in das erste Volumen (1, 2) zu pumpen.

5. Vorrichtung nach einem der vorhergehenden Ansprüche,
**dadurch gekennzeichnet,**
**dass** die Vorrichtung (10) ein drittes Ventil (11) umfasst, mit welchem die Pumpe (3) eingangsseitig mit dem zu entgasenden Entgasungsgefäß (7) verbunden ist.

6. Vorrichtung nach Anspruch 5,
**dadurch gekennzeichnet,**
**dass** die Vorrichtung (10) ausgestaltet ist, um mittels der Steuereinrichtung (19) die Ventilmittel (12) derart zu schalten, dass die Ventilmittel (12) die Pumpe (3) ausgangsseitig nur mit dem Luftauslass (22) verbinden, um das erste Ventil (15), das zweite Ventil (14) und das dritte Ventil (11) zu öffnen und um anschließend mittels der Pumpe (3) das Entgasungsgefäß (7), das erste Volumen (1, 2) und das zweite Volumen (6) zu evakuieren.

7. Vorrichtung nach Anspruch 5 oder Anspruch 6,
**dadurch gekennzeichnet,**
**dass** die Vorrichtung (10) ausgestaltet ist, um mittels der Steuereinrichtung (19) die Ventilmittel (12) derart zu schalten, dass die Ventilmittel (12) die Pumpe (3) ausgangsseitig nur mit dem ersten Volumen (1, 2) verbinden, um das erste Ventil (15) und das zweite Ventil (14) zu öffnen und um das dritte Ventil (11) zu schließen, so dass anschließend ein Druckausgleich in einem Gesamtvolumen, welches von dem ersten Volumen (1, 2) und dem zweiten Volumen (6) sowie einem Totvolumen (22) gebildet wird, ausgeführt wird.

8. Vorrichtung nach einem Ansprüche 5 bis 7,
**dadurch gekennzeichnet,**
**dass** die Vorrichtung (10) ausgestaltet ist, um mittels der Steuereinrichtung (19) die Ventilmittel (12) derart zu schalten, dass die Ventilmittel (12) die Pumpe (3) ausgangsseitig nur mit dem ersten Volumen (1, 2) verbinden, um das erste Ventil (15) und das zweite Ventil (14) zu schließen, um das dritte Ventil (11) zu öffnen und um anschließend mittels der Pumpe (3) Gas von der Einrichtung (7) in das erste Volumen (1, 2) zu pumpen.

9. Vorrichtung nach einem der vorhergehenden Ansprüche,
**dadurch gekennzeichnet,**
**dass** die Vorrichtung (10) einen Sensor (4) umfasst, welcher in dem ersten Volumen (1) angeordnet ist, und
**dass** der Sensor (4) ausgestaltet ist, um das Gas in dem ersten Volumen (1) zu analysieren.

10. Vorrichtung nach einem der vorhergehenden Ansprüche,
**dadurch gekennzeichnet,**
**dass** das erste Volumen ein erstes Teilvolumen (1) und ein zweites Teilvolumen (2) umfasst, welche über ein weiteres Ventil (13) der Vorrichtung (10) verbunden sind, dass die Vorrichtung (10) ausgestaltet ist, um mittels der Steuereinrichtung (19) die Ventilmittel (12) derart zu schalten, dass die Ventilmittel (12) die Pumpe (3) ausgangsseitig nur mit dem ersten Volumen (1, 2) verbinden, um das erste Ventil (15), das dritte Ventil (11) und das weitere Ventil (13) zu schließen, um das zweite Ventil (14) zu öffnen und um anschließend mittels der Pumpe (3) Gas von dem zweiten Teilvolumen (2) in das erste Teilvolumen (1) zu pumpen.

11. Vorrichtung nach einem der vorhergehenden Ansprüche,
**dadurch gekennzeichnet,**
**dass** die Vorrichtung (10) zur Entgasung einer Isolierflüssigkeit einer Hochspannungsanlage (40) ausgestaltet ist.

12. Prüfsystem,
wobei das Prüfsystem (30) eine Auswerteeinheit (20) und ein Vorrichtung (10) nach einem der vorhergehenden Ansprüche umfasst, um eine Analyse des Gases in oder von dem Entgasungsgefäß (7) durchzuführen.

13. Verfahren zur Entgasung eines Entgasungsgefäßes (7), wobei das Verfahren die folgenden Schritten umfasst:
Verbinden eines ersten Volumens (1, 2) mit einem separat von dem ersten Volumen (1, 2) vorgesehenen zweiten Volumen (6),
Evakurieren des Entgasungsgefäßes (7), des ersten Volumens (1, 2) und des zweiten Volumens (6),
Abtrennen der Einrichtung (7) von dem ersten Volumen (1, 2) und dem zweiten Volumen (6),
Durchführen eines Druckausgleichs in einem Gesamtvolumen, welches von dem ersten Volumen (1, 2) und dem zweiten Volumen (6) sowie einem Totvolumen (22) gebildet wird,
Abtrennen des zweiten Volumens (6) von dem ersten Volumen (1, 2),
Befüllen des Entgasungsgefäßes (7) mit einer zu entgasenden Flüssigkeit, und
Entgasen des Entgasungsgefäßes (7) in das erste Volumen (1, 2).

14. Verfahren nach Anspruch 13,
**dadurch gekennzeichnet,**
**dass** das erste Volumen ein erstes Teilvolumen (1) und ein zweites Teilvolumen (2) umfasst, und
**dass** nach dem Entgasen des Entgasungsgefäßes (7) folgende Schritte durchgeführt werden:
Abtrennen des zweiten Teilvolumens (2) von dem ersten Teilvolumen (1), und
Pumpen eines Gases aus dem zweiten Teilvolumen (2) in das erste Teilvolumen (1).

15. Verfahren nach Anspruch 13 oder Anspruch 14,
**dadurch gekennzeichnet,**
**dass** das Verfahren mit einer Vorrichtung (10) nach einem der Ansprüche 1-12 ausgeführt wird.

## Claims

1. An apparatus for degassing a degassing vessel (7) which can be filled with a liquid to be degassed,
having a pump (3), which is designed for pumping gas and which, at a discharge end, is connected to an air outlet (21) via valve means (12) and to a first volume (1, 2) via the valve means (12),
wherein the first volume (1, 2) and a second volume (6) are connected via a first valve (15),
wherein, at an intake end, the pump (3) is connected to the first volume (1, 2) via a second valve (14) and can be connected to the degassing vessel (7) to be degassed, and
having a control unit (19) for activating the pump (3), the valve means (12), the first valve (15) and the second valve (14),
wherein the apparatus (10) is designed in order to switch the valve means (12) by means of the control unit (19) such that the valve means (12) connect the pump (3) only to the air outlet (22) at the discharge end, in order to open the first valve (15) and the second valve (14) and to then evacuate the degassing vessel (7), the first volume (1, 2) and the second volume (6) by means of the pump (3).

2. The apparatus according to claim 1,
**characterized in that**
the second volume (6) is at least ten times larger than the first volume (1, 2).

3. The apparatus according to one of the preceding claims,
**characterized in that**
the apparatus (10) is designed in order to switch the valve means (12) by means of the control unit (19) such that the valve means (12) connect the pump (3) only to the first volume (1, 2) at the discharge end, and in order to open the first valve (15) and the second valve (14) such that a pressure equalisation is then carried out in an overall volume, which is formed by the first volume (1, 2) and the second volume (6) and also a dead volume (22).

4. The apparatus according to one of the preceding claims,
**characterized in that**
the apparatus (10) is designed in order to switch the valve means (12) by means of the control unit (19) such that the valve means (12) connect the pump (3) only to the first volume (1, 2) at the discharge end and in order to close the first valve (15) and the second valve (14) and to then pump gas from the device (7) into the first volume (1, 2) by means of the pump (3).

5. The apparatus according to one of the preceding claims,
**characterized in that**
the apparatus (10) comprises a third valve (11), with which, at the intake end, the pump (3) is connected to the degassing vessel (7) to be degassed.

6. The apparatus according to claim 5,
**characterized in that**
the apparatus (10) is designed in order to switch the valve means (12) by means of the control unit (19) such that the valve means (12) connect the pump (3) only to the air outlet (22) at the discharge end, in order to open the first valve (15), the second valve (14) and the third valve (11) and to then evacuate the degassing vessel (7), the first volume (1, 2) and the second volume (6) by means of the pump (3).

7. The apparatus according to claim 5 or claim 6,
**characterized in that**
the apparatus (10) is designed in order to switch the valve means (12) by means of the control unit (19) such that the valve means (12) connect the pump (3) only to the first volume (1, 2) at the discharge end, in order to open the first valve (15) and the second valve (14) and in order to close the third valve (11), such that a pressure equalisation is then performed in an overall volume that is formed by the first volume (1, 2) and the second volume (6) and also a dead volume (22).

8. The apparatus according to any one of Claims 5 to 7,
**characterized in that**
the apparatus (10) is designed in order to switch the valve means (12) by means of the control unit (19) such that the valve means (12) connect the pump (3) only to the first volume (1, 2) at the discharge end, in order to close the first valve (15) and the second valve (14), in order to open the third valve (11) and to then pump gas from the device (7) into the first volume (1, 2) by means of the pump (3).

9. The apparatus according to one of the preceding claims
**characterized in that**
the apparatus (10) comprises a sensor (4), which is arranged in the first volume (1), and
the sensor (4) is designed in order to analyze the gas in the first volume (1).

10. The apparatus according to one of the preceding claims,
**characterized in that**
the first volume comprises a first partial volume (1) and a second partial volume (2) that are connected via an additional valve (13) of the apparatus (10),
the apparatus (10) is designed in order to switch the valve means (12) by means of the control unit (19) such that the valve means (12) connect the pump (3) only to the first volume (1, 2) at the discharge end, in order to close the first valve (15), the third valve (11) and the additional valve (13), in order to open the second valve (14) and to then pump gas from the second partial volume (2) into the first partial volume (1) by means of the pump (3).

11. The apparatus according to one of the preceding claims,
**characterized in that**
the apparatus (10) is designed for degassing an insulating fluid of a high-voltage installation (40).

12. A test system,
wherein the test system (30) comprises an evaluation unit (20) and an apparatus (10) according to one of the preceding claims, in order to carry out an analysis of the gas in or from the degassing vessel (7).

13. A method of degassing a degassing vessel (7), the method comprising the following steps:
connecting a first volume (1, 2) to a second volume (6) which is supplied separately from the first volume (1, 2),
evacuating the degassing vessel (7), the first volume (1, 2) and the second volume (6),
cutting the device (7) off from the first volume (1, 2) and the second volume (6),
performing a pressure equalisation in an overall volume which is formed by the first volume (1, 2) and the second volume (6) and also a dead volume (22),
cutting the second volume (6) off from the first volume (1, 2),
filling the degassing vessel (7) with a liquid to be degassed, and
degassing the degassing vessel (7) into the first volume (1, 2).

14. The method according to Claim 13,
**characterized in that**
the first volume comprises a first partial volume (1) and a second partial volume (2), and
after the degassing of the degassing vessel (7), the following steps are carried out:
cutting the second partial volume (2) off from the first partial volume (1), and
pumping a gassing out of the second partial volume (2) into the first partial volume (1).

15. The method according to Claim 13 or claim 14,
**characterized in that**
the method is performed with an apparatus (10) according to one of Claims 1-12.

## Revendications

1. Dispositif de dégazage d'un récipient de dégazage (7) pouvant être rempli d'un liquide à dégazer,
comportant une pompe (3) qui est conçue pour pomper du gaz et qui est reliée côté sortie d'une part à une sortie d'air (21) par l'intermédiaire de moyens de soupape (12) et d'autre part à un premier volume (1, 2) par l'intermédiaire des moyens de soupape (12),
dans lequel le premier volume (1, 2) et un second volume (6) sont reliés par l'intermédiaire d'une première soupape (15),
dans lequel la pompe (3) est reliée côté entrée d'une part au premier volume (1, 2) par l'intermédiaire d'une deuxième soupape (14) et peut être reliée d'autre part au récipient de dégazage (7) à dégazer, et
comportant un appareil de commande (19) permettant de commander la pompe (3), les moyens de soupape (12), la première soupape (15) et la deuxième soupape (14),
dans lequel le dispositif (10) est conçu pour commuter les moyens de soupape (12) à l'aide de l'appareil de commande (19) de telle sorte que les moyens de soupape (12) ne relient la pompe (3), côté sortie, qu'à la sortie d'air (22) pour ouvrir la première soupape (15) et la deuxième soupape (14) et pour évacuer ensuite, à l'aide de la pompe (3), le récipient de dégazage (7), le premier volume (1, 2) et le second volume (6).

2. Dispositif selon la revendication 1,
**caractérisé en ce**
**que** le second volume (6) est au moins dix fois plus grand que le premier volume (1, 2).

3. Dispositif selon l'une des revendications précédentes,
**caractérisé en ce**
**que** le dispositif (10) est conçu pour commuter les moyens de soupape (12) à l'aide du dispositif de commande (19) de telle sorte que les moyens de soupape (12) ne relient la pompe (3), côté sortie, qu'au premier volume (1, 2), et pour ouvrir la première soupape (15) et la deuxième soupape (14) de sorte qu'une compensation de pression est ensuite effectuée dans un volume total qui est formé par le premier volume (1, 2) et le second volume (6) ainsi que par un volume mort (22).

4. Dispositif selon l'une des revendications précédentes,
**caractérisé en ce**
**que** le dispositif (10) est conçu pour commuter les moyens de soupape (12) à l'aide du dispositif de commande (19) de telle sorte que les moyens de soupape (12) ne relient la pompe (3), côté sortie, qu'au premier volume (1, 2), et pour fermer la première soupape (15) et la deuxième soupape (14) et pour pomper ensuite du gaz de l'appareil (7) au premier volume (1, 2) à l'aide de la pompe (3).

5. Dispositif selon l'une des revendications précédentes,
**caractérisé en ce**
**que** le dispositif (10) comprend une troisième soupape (11) au moyen de laquelle la pompe (3) est reliée côté entrée au récipient de dégazage (7) à dégazer.

6. Dispositif selon la revendication 5,
**caractérisé en ce**
**que** le dispositif (10) est conçu pour commuter les moyens de soupape (12) à l'aide de l'appareil de commande (19) de telle sorte que les moyens de soupape (12) ne relient la pompe (3), côté sortie, qu'à la sortie d'air (22) pour ouvrir la première soupape (15), la deuxième soupape (14) et la troisième soupape (11) et pour évacuer ensuite, à l'aide de la pompe (3), le récipient de dégazage (7), le premier volume (1, 2) et le second volume (6).

7. Dispositif selon la revendication 5 ou la revendication 6,
**caractérisé en ce**
**que** le dispositif (10) est conçu pour commuter les moyens de soupape (12) à l'aide du dispositif de commande (19) de telle sorte que les moyens de soupape (12) ne relient la pompe (3), côté sortie, qu'au premier volume (1, 2), pour ouvrir la première soupape (15) et la deuxième soupape (14) et pour fermer la troisième soupape (11) de sorte qu'une compensation de pression est ensuite effectuée dans un volume total qui est formé par le premier volume (1, 2) et le second volume (6) ainsi que par un volume mort (22).

8. Dispositif selon l'une des revendications 5 à 7,
**caractérisé en ce**
**que** le dispositif (10) est conçu pour commuter les moyens de soupape (12) à l'aide du dispositif de commande (19) de telle sorte que les moyens de soupape (12) ne relient la pompe (3), côté sortie, qu'au premier volume (1, 2), pour fermer la première soupape (15) et la deuxième soupape (14), pour ouvrir la troisième soupape (11) et pour pomper ensuite du gaz de l'appareil (7) au premier volume (1, 2) à l'aide de la pompe (3).

9. Dispositif selon l'une des revendications précédentes,
**caractérisé en ce**
**que** le dispositif (10) comprend un capteur (4) qui est disposé dans le premier volume (1), et
**que** le capteur (4) est conçu pour analyser le gaz dans le premier volume (1).

10. Dispositif selon l'une des revendications précédentes,
**caractérisé en ce**
**que** le premier volume comprend un premier volume partiel (1) et un second volume partiel (2), qui sont reliés par l'intermédiaire d'une autre soupape (13) du dispositif (10), **que** le dispositif (10) est conçu pour commuter les moyens de soupape (12) à l'aide du dispositif de commande (19) de telle sorte que les moyens de soupape (12) ne relient la pompe (3), côté sortie, qu'au premier volume (1, 2), pour fermer la première soupape (15), la troisième soupape (11) et l'autre soupape (13), pour ouvrir la deuxième soupape (14) et pour pomper ensuite du gaz du second volume partiel (2) au premier volume partiel (1) à l'aide de la pompe (3).

11. Dispositif selon l'une des revendications précédentes,
**caractérisé en ce**
**que** le dispositif (10) est conçu pour dégazer un liquide isolant d'une installation à haute tension (40).

12. Système de contrôle,
dans lequel le système de contrôle (30) comprend une unité d'évaluation (20) et un dispositif (10) selon l'une des revendications précédentes pour effectuer une analyse du gaz dans ou à partir du récipient de dégazage (7).

13. Procédé de dégazage d'un récipient de dégazage (7), dans lequel le procédé comprend les étapes suivantes :
liaison d'un premier volume (1, 2) à un second volume (6) prévu séparément du premier volume (1, 2),
évacuation du récipient de dégazage (7), du premier volume (1, 2) et du second volume (6),
séparation de l'appareil (7) du premier volume (1, 2) et du second volume (6),
réalisation d'une compensation de pression dans un volume total formé par le premier volume (1, 2) et le second volume (6) ainsi que par un volume mort (22),
séparation du second volume (6) du premier volume (1, 2),
remplissage du récipient de dégazage (7) avec un liquide à dégazer, et
dégazage du récipient de dégazage (7) dans le premier volume (1, 2).

14. Procédé selon la revendication 13,
**caractérisé en ce**
**que** le premier volume comprend un premier volume partiel (1) et un second volume partiel (2), et
**que** les étapes suivantes sont réalisées après le dégazage du récipient de dégazage (7) :
séparation du second volume partiel (2) du premier volume partiel (1), et
pompage d'un gaz du second volume partiel (2) au premier volume partiel (1).

15. Procédé selon la revendication 13 ou la revendication 14,
**caractérisé en ce**
**que** le procédé est mis en oeuvre au moyen d'un dispositif (10) selon l'une des revendications 1 à 12.
